# EUROPEAN PATENT APPLICATION

(11) **EP 4 712 087 A1**
(43) Date of publication of application: **18.03.2026**
(21) Application number: 24200303.6
(22) Date of filing: 13.09.2024
(51) Int. Cl.: G16C 20/00

(54) **CLASSIFYING MOLECULAR STRUCTURES INTO EQUIVALENT SETS**

(71) Applicant: HQS Quantum Simulations GmbH, 76131 Karlsruhe (DE)
(72) Inventor: Hodecker, Manuel, 69126 Heidelberg (DE); Covito, Fabio, 20255 Hamburg (DE); Pinski, Peter, 76149 Karlsruhe (DE)
(74) Representative: Durm Patentanwälte PartG mbB

(57) **Abstract**

The present invention relates to an apparatus (10) for classifying a plurality of molecular structures (20) into a plurality of equivalent sets (30), comprising: an interface (60) for receiving input data (70) relating to the molecular structures (20) to be classified and at least one tolerance parameter for setting a tolerance, a processing unit (80) for generating a plurality of graphs (100) from the plurality of molecular structures (20), comparing (120) each individual graph from the plurality of graphs (100) to all other individual graphs, determining whether the graphs are equivalent or non-equivalent within the tolerance based on the tolerance parameter, and classifying the graphs (100) into the plurality of equivalent sets (30), wherein each individual equivalent set of the plurality of equivalent sets (30) is non-equivalent to all other equivalent sets (30). The present invention further relates to a method for classifying a plurality of molecular structures (20) into a plurality of equivalent sets (30), a computer program, a data carrier signal and a computer-readable medium.

## Description

The present invention relates to an apparatus for classifying a plurality of molecular structures into a plurality of equivalent sets, for example for simulating nuclear magnetic resonance (NMR) spectra and/or thermodynamic properties and/or chemical reactions. The present invention further relates to a method, a computer program, a data carrier signal and a computer-readable medium.

Molecules typically do not maintain a rigid three-dimensional structure but instead exist in multiple conformations, especially at ambient temperature and ambient pressure and in liquid state or gaseous state or in solution. These conformations represent different spatial arrangements of the same molecule that interconvert rapidly at ambient conditions without the breaking of chemical bonds, most commonly due to internal rotation around single bonds. In other words, conformations are different structural forms of a molecule with identical atom composition and connectivity, differing only in the relative orientations of their molecular parts, such as for example functional groups, and the resulting interatomic distances. The existence of multiple stable conformations - referred to as conformers - at ambient conditions, makes their accurate modeling essential for predicting or interpreting experimental outcomes, such as simulating or assigning spectroscopic data, especially NMR spectra, modeling chemical reactions, or calculating thermodynamic properties.

In this context conformers and rotamers are distinguished from each other. Conformers are distinct structures that differ in features such as interatomic distances or torsional angles, whereas rotamers are equivalent structures that differ only by the permutation of identical atoms, typically by rotations around single bonds. For example, in an organic molecule containing a methyl group, rotamers are generated by 120° rotations of the methyl group around its connecting single bond, leading to indistinguishable structures, in particular with the same bond topologies.

Current conformer search software, such as RDKit^{™} (RDKit: Open-source cheminformatics. https://rdkit.org) and CREST^{™} (P. Pracht et al.; J. Chem. Phys. 2024, 160, 114110; DOI: 10.1063/5.0197592; https://github.com/crest-lab), provides functionality to generate and analyze different conformations including both conformers and rotamers of a given molecular structure input. These tools, however, do not provide the capability to sort a given set of molecular structures purely based on their internal coordinates and determine their equivalence or non-equivalence. This limitation complicates the accurate accounting of all possible conformers and rotamers, which is particularly crucial in spectroscopic predictions and analyses.

In view of this, it is an object of the present invention to provide an improved apparatus for classifying a plurality of molecular structures into a plurality of equivalent sets.

In a first aspect, the present invention relates to an apparatus for classifying a plurality of molecular structures into a plurality of equivalent sets, for example for simulating NMR spectra and/or thermodynamic properties and/or chemical reactions, comprising:
an interface for receiving input data relating to the molecular structures to be classified and at least one tolerance parameter for setting a tolerance,
a processing unit for
   - generating a plurality of graphs from the plurality of molecular structures, wherein preferably for each individual molecular structure of the plurality of molecular structures one graph is generated,
   - comparing each individual graph from the plurality of graphs to all other individual graphs,
   - determining whether the graphs are equivalent or non-equivalent within the tolerance based on the tolerance parameter, and
   - classifying the graphs into the plurality of equivalent sets, wherein each individual equivalent set of the plurality of equivalent sets is non-equivalent to all other equivalent sets.

In a second aspect, the present invention relates to a method for classifying a plurality of molecular structures into a plurality of equivalent sets, for example for simulating NMR spectra and/or thermodynamic properties and/or chemical reactions, comprising steps of:
- receiving input data relating to the molecular structures to be classified and at least one tolerance parameter for setting a tolerance,
- generating a plurality of graphs from the plurality of molecular structures, wherein preferably for each individual molecular structure of the plurality of molecular structures one graph is generated,
- comparing each individual graph from the plurality of graphs to all other individual graphs,
- determining whether the graphs are equivalent or non-equivalent within the tolerance based on the tolerance parameter, and
- classifying the graphs into the plurality of equivalent sets, wherein each individual equivalent set of the plurality of equivalent sets is non-equivalent to all other equivalent sets.

In a third aspect, the present invention relates to a computer program comprising instructions which, when the program is executed by a computer, cause the computer to carry out the method according to the previous aspect of the invention.

In a fourth aspect, the present invention relates to a data carrier signal carrying the computer program according to the previous aspect of the invention.

In a fifth aspect, the present invention relates to a computer-readable medium comprising instructions which, when executed by a computer, cause the computer to carry out the method of the invention.

A first preferred aspect relates to an apparatus for completing a set of permutations representing a plurality of equivalent sets of molecular structures, for example for simulating NMR spectra and/or thermodynamic properties and/or chemical reactions, comprising:
- an interface for receiving input data relating to an incomplete set of permutations,
a processing unit for
(a) converting the incomplete set of permutations into a plurality of permutation matrices, wherein each permutation matrix preferably represents one individual molecular structure,
(b) generating a new permutation matrix by multiplying one individual permutation matrix of the plurality of permutation matrices with another individual permutation matrix of the plurality of permutation matrices or with itself,
(c) comparing the new permutation matrix with the plurality of permutation matrices for determining if the new permutation matrix is identical or not identical to any other individual permutation matrix of the plurality of permutation matrices,
(d) including the new permutation matrix into the plurality of permutation matrices, if the new permutation matrix is not identical to any other individual permutation matrix of the plurality of permutation matrices,
(e) repeating (b) to (d), at least until all possible different multiplications within the plurality of permutation matrices, including the multiplications of individual permutation matrices with themselves, have been performed.

A second preferred aspect relates to a method for completing a set of permutations representing a plurality of equivalent sets of molecular structures, for example for simulating NMR spectra and/or thermodynamic properties and/or chemical reactions, comprising steps of:
- receiving input data relating to an incomplete set of permutations,
   (a) generating a new permutation matrix by multiplying one individual permutation matrix of the plurality of permutation matrices with another individual permutation matrix of the plurality of permutation matrices or with itself,
   (b) multiplying two individual permutation matrices of the plurality of permutation matrices which each other to generate a new permutation matrix,
   (c) comparing the new permutation matrix with the plurality of permutation matrices for determining if the new permutation matrix is identical or not identical to any other individual permutation matrix of the plurality of permutation matrices,
   (d) including the new permutation matrix into the plurality of permutation matrices if the new permutation matrix is not identical to any other individual permutation matrix of the plurality of permutation matrices,
   (e) repeating (b) to (d), at least until all possible different multiplications within the plurality of permutation matrices, including the multiplications of individual permutation matrices with themselves, have been performed.

A third preferred aspect relates to a computer program comprising instructions which, when the program is executed by a computer, cause the computer to carry out the method according to the previous preferred aspect.

A fourth preferred aspect relates to a data carrier signal carrying the computer program according to the previous preferred aspect.

A fifth preferred aspect relates to a computer-readable medium comprising instructions which, when executed by a computer, cause the computer to carry out the method of the second preferred aspect.

Preferred embodiments of the invention are defined in the dependent claims. It shall be understood that the claimed apparatus, method, computer program, data carrier signal and computer-readable medium have similar and/or identical preferred embodiments as the claimed apparatus or the claimed method, and in particular as defined in the dependent claims and as disclosed herein.

The apparatus is preferably configured to carry out the method for classifying a plurality of molecular structures and the method for completing a set of permutations, more preferably in consecutive order. In other words, the apparatus for classifying a plurality of molecular structures and the apparatus for completing a set of permutations are preferably identical and more preferably share identical or at least similar preferred embodiments. Consequently, all features of the apparatus for classifying a plurality of molecular structures can be applied for the apparatus for completing a set of permutations, either in isolation or in combination. Likewise, all features of the apparatus for completing a set of permutations can be applied to the apparatus for classifying a plurality of molecular structures, either in isolation or in combination. The same considerations apply for the method for classifying a plurality of molecular structures and the method for completing a set of permutations. It should be noted that the plurality of equivalent sets obtained after classifying the graphs may in particular serve as the incomplete set of permutations received as input data for completing the set of permutations. Advantageously, after completing, a completed set of permutations represents a completed plurality of equivalent sets of molecular structures, which may preferably be utilized for the simulation of NMR spectra and/or thermodynamic simulations and/or chemical reactions.

The present invention is based on the idea of applying graph theory for the identification of molecular structures that are equivalent to each other and therefore in particular rotamers and of molecular structures that are non-equivalent and therefore in particular conformers. In comparison to previous approaches, the approach of the present invention is further based on the idea, that the classification of molecular structures is performed preferably merely based on the atom composition of the molecular structures and their structural parameters such as bond lengths, bond angles, and dihedral angles. In particular, the invention thus provides an atom mapping that defines the permutation of atoms between rotamers.

The present invention provides an advantageous approach for identifying and sorting equivalent molecular structure geometries and non-equivalent molecular structure geometries. Different permutations can particularly correspond to different rotamers, i.e. different permutations of atoms. The molecular structures are in particular 3D molecular structures. This automated classification approach can also be regarded as a sorting process, along with the identification of atom permutations. In particular, the permutations are determined using the isomorphisms. Favorably, the current approach can enhance the efficiency of chemical simulation workflows, particularly those related to NMR spectroscopy, resulting in more accurate predictions of NMR spectra, in better alignment with experimental data. Advantageously, this allows for an automated and robust classification of molecular structures into equivalent sets and non-equivalent sets, which can be used for an efficient simulation.

The apparatus can particularly be implemented in the form of a computer or a software running on a computer. It is to be understood that the full functionality of the apparatus can be provided by a software, preferably a scientific simulation software. In other words, the apparatus itself may particularly be implemented in the form of software. In case the apparatus is implemented in form of software, this software corresponds to the method, which is preferably a computer implemented method. The different interfaces and units can be implemented in the form of hard- and/or software and can also be combined with one another upon need. The apparatus / scientific simulation software can particularly be a scientific simulation tool for simulating NMR spectra or thermodynamic properties. The apparatus / scientific simulation software can also be employed for simulating chemical reactions of different components and their behavior under changing or varying external conditions. The interface preferably interacts with the processing unit. In particular, this interaction can be performed either via a wired connection between the interface and the processing unit or via an internet connection. The interface may thus particularly correspond to an interface for interacting with another computer. In particular, the interface to an internet connection may be included in the interface. The interface allows processing a receiving input data that may be already present or that may be received from another entity, e. g. via the internet. In addition, it is possible that the processing unit directly interacts with the scientific simulation software. The processing unit may thus directly communicate with the scientific simulation software. The simulation result provided by the scientific simulation software can then be fed back to a user.

In a preferred embodiment, the processing unit is configured for classifying the graphs into the plurality of equivalent sets, wherein each individual equivalent set of the plurality of equivalent sets comprises either one single graph or a plurality of graphs representing rotamer molecular structures. The classification is advantageously achieved by generating a new equivalent set for each individual graph, that is not identical to all other individual graphs from the plurality of graphs and preferably assigning this individual graph to the newly generated equivalent set. The classification is preferably achieved by grouping together equivalent graphs. In other words, if one individual graph is identical to another individual graph, these graphs are in particular grouped together in one equivalent set and thereby said individual graphs are more preferably mapped together.

Especially for the simulation of NMR spectra, proper identification and averaging over rotamers is critical. Current tools do not guarantee this accuracy, as the assignment of conformers and rotamers is at least partly flawed, and the ensemble generated is incomplete. An incomplete atom mapping between rotamers can lead to incorrect averaging of spectral parameters, compromising the accuracy of spectroscopic interpretations.

In a preferred embodiment, the processing unit is configured for classifying the graphs into the plurality of equivalent sets, wherein each individual equivalent set of the plurality of equivalent sets comprises either one single graph or a plurality of graphs representing molecular structures with equivalent bond topologies.

In particular, bonds between atoms of the molecules are first identified using a distance criterion, and then graphs, in particular chemical graphs are generated from this information. For the generation of the graphs, the Python library NetworkX^{®} (networkx.github.io) may be employed. In particular, the nodes of the graphs represent atoms with their chemical element symbols included in node labels. The bonds between atoms are preferably represented as edges, with the structural parameters included in edge labels.

The generation of the graphs can be illustrated by the following non-limiting example of a carbon dioxide molecule (CO₂):
Initial molecular structure: CO₂
Input molecular structure:

| | |
|---|---|
| Indices | 0 1 2 |
| Species | O=C=O |
| Distances | -d₁-\|-d₂- |
| Angles | - 180°- |

The chemical graph of CO₂ is generated such, that the nodes including the atom species for the respective indices and position data are stored as follows:

```
       [
               (0, {'species': 'O', 'position': (-1.399642, -0.239115, 0.0)}),
               (1, {'species': 'C', 'position': (-0.0, -0.116517, 0.0)}),
               (2, {'species': 'O', 'position': (1.399642, 0.006078, 0.0)}),
       ],
```

the edges including the indices and the structural data are stored as follows:

```
       [
               (0, 1, {'distance': {(0, 1): 1.40500106682}}),
               (0, 2, {'angle': {(0, 1, 2): 3.14159052648}}),
               (1, 2, {'distance': {(1, 2): 1.40500080505}}),
       ].
```

In a preferred embodiment, the processing unit is configured for classifying the graphs into the plurality of equivalent sets, wherein each individual equivalent set of the plurality of equivalent sets is representing a non-equivalent conformer molecular structure in comparison to the molecular structures of all other equivalent sets. In other words, the molecular structures represented by the graphs sorted in one equivalent set are isomorphic rotamers, while the molecular structures represented by the graphs of two different equivalent sets are conformers. Advantageously, the equivalent set of isomorphic structures provide a direct atom mapping between the equivalent structures.

In a preferred embodiment, the interface is configured for receiving input data relating to the molecular structures, wherein the input data comprises an atom composition and structural parameters such as bond lengths and/or bond angles and/or dihedral angles. Advantageously this input data is sufficient and no other parameters such as energy and/or rotational constants and/or Cartesian root mean square deviations are required. However, in the alternative, it is also possible to include other parameters such as the energy and/or rotational constants and/or Cartesian root mean square deviations.

In a preferred embodiment, the interface is configured for receiving input data including atomic coordinates of each atom of the molecular structures. The processing unit is configured for generating the plurality of graphs from the plurality of molecular structures by determining a molecular geometry representation with bond lengths, angles and dihedrals based on the atomic coordinates (can also be referred to as internal coordinates). An efficient implementation can be obtained.

In a preferred embodiment, the processing unit is configured for comparing each individual graph from the plurality of graphs to all other individual graphs based on a determination whether node and edge topologies match and the graphs are isomorphic. The processing unit is preferably configured for removing falsely identified isomorphisms. An efficient implementation can be obtained.

In a preferred embodiment, the processing unit is configured for determining whether the graphs are equivalent or non-equivalent based on a selection of isomorphisms. An efficient implementation can be obtained.

In a preferred embodiment, the apparatus comprises a conformation generating unit for generating input data relating to the molecular structures. Preferably, the molecular structures comprise a plurality of non-equivalent conformer molecular structures and rotamer molecular structures. The conformation generating unit is preferably realized by the Python library NetworkX^{®}.

In a preferred embodiment, the interface comprises a computer interface and a user interface. The user interface is preferably configured for receiving an initial molecular structure. The computer interface is favorably configured for receiving the input data relating to the molecular structures. The interface may advantageously allow interaction with the user. For this, the interface may comprise the user interface, which may particularly correspond to a software interface that allows the user to provide input, especially an initial molecular structure. Preferably, the user can provide the input in the form of the initial chemical structure as text, in particular natural language, or data from an experimental measurement, such as X-ray diffraction data. It may also be possible that the user provides input in the form of text or graphical illustrations, e.g., a graphical illustration from a scientific paper etc. Advantageously, an easy-to-use user interface is provided. Also, the time for new users to get started with the scientific simulation software can be reduced by the straightforward input of initial molecular structures

Beneficially, the conformation generating unit is configured for generating the input data relating to the molecular structures from the initial molecular structure. The initial molecular structure can be for example entered by a molecule editor such as ChemDraw^{®} or ChemSketch^{™}. The initial molecular structure may be provided as a 2D molecular structure in a so-called SMILES string. Moreover, the initial molecular structure may be provided as a name associated with the molecular structure. In this case the SMILES string is preferably obtained via a database such as for example the PubChem^{®} database. The 2D molecular structure is preferably converted into an initial 3D molecular structure, in particular by using the RDKit^{™} or Open Babel^{™} software. The initial 3D molecular structure is beneficially used as a starting point for generating the input data relating to the plurality of molecular structures. Optionally, an initial geometry optimization of the 3D molecular structure may be performed. Next, CREST^{™} may be used to generate the input data relating to the plurality of molecular structures, wherein in particular the only the data relating to the molecular structures is used, while the assignment of conformers and rotamers provided by CREST^{™} is preferably ignored.

In a preferred embodiment, the user interface is configured for receiving the initial molecular structure from a scientific measurement, such as preferably as X-ray diffraction data.

In a preferred embodiment, the interface is configured for receiving the tolerance parameter relating to a bond length tolerance and/or a bond angle tolerance and/or a dihedral angle tolerance. Favorably, the bond length tolerance and/or the bond angle tolerance and/or the dihedral angle tolerance are each from 0.1% to 10%, preferably from 0.1% to 5%, more preferably from 1% to 3%. With respect to the angles, the relative tolerance value may refer to a 180° value or to the current value.

In a preferred embodiment, the interface is configured for receiving the tolerance parameter, wherein the bond length tolerance is from 1 pm to 10 pm, preferably from 1 pm to 5 pm, more preferably from 2 pm to 3 pm and/or the bond angle tolerance and/or the dihedral angle tolerance are each from 0.1° to 10°, preferably from 0.2° to 5°, more preferably 0.5°.

In particular, the input molecular structures are provided in a XYZ format, which can be generated by a conformer search software, e.g., by RDKit^{™} or by CREST^{™}, or by performing a molecular dynamics simulation. Preferably, the XYZ format includes the atom composition and the molecular structure parameters as coordinates, which are then converted into the molecular graphs. Subsequently, a first individual graph from the plurality of graphs is taken as a reference and compared to all other graphs of the plurality of graphs. If all structural parameters such as for example the bond lengths, the bond angles, and the dihedral angles are within the afore set tolerance, the structures are determined as equivalent, in particular irrespective of the atom ordering in the XYZ input. If the structures are equivalent, all possible isomorphisms, such as for instance permutations needed to map one structure to the other, are returned classified together in an equivalent set, in particular with the respective index of the molecular structures. This procedure is preferably repeated with the remaining graphs from the plurality of graphs. This loop can continue as long as required. The equivalent molecular structures are beneficially classified again into the respective equivalent sets together with their isomorphisms, and the remaining molecular structures are compared again, until all graphs and thus all molecular structures are completely classified and sorted into the equivalent sets.

Favorably, one individual graph, representing one molecular structure is chosen as a representative molecular structure for each equivalent set. In particular, the molecular structures that were identified as equivalent are collected, preferably with their reference and their isomorphisms or rotamers. As a result, the non-equivalent conformer molecular structures are advantageously grouped together with the permutational atom mappings for their rotamers. The output valuably provides a plurality of equivalent sets, wherein the equivalent sets represent non-equivalent and thus distinguishable molecular structures. One distinct equivalent set beneficially comprises one or a plurality of molecular structures, with each of those having a list of isomorphisms representing rotamers. Preferably, the current invention thus gives access to the number of equivalent structures per conformer and the atom mapping of one rotamer to another. If provided in the input data, energetic information of the different molecular structures is preserved. The output is for example compatible with the data validation Python package Pydantic^{®} (https://docs.pydantic.dev/latest/) and other standard formats. The output may be validated by the Python package Pydantic^{®}.

In a preferred embodiment, the apparatus comprises an NMR spectrum simulation unit for simulating an NMR spectrum from the plurality of equivalent sets. Preferably, after completing the set of permutations, in particular after completing the plurality of equivalent sets, preferably representing an ensemble of conformer and rotamer molecular structures, quantum-chemical calculations are performed for each individual equivalent set to obtain energies and NMR parameters. Favorably, the NMR parameters are averaged over the molecular structures comprised by one individual equivalent set, in particular over all rotamers comprised within one equivalent set. Beneficially, the different conformers are weighted according to their Boltzmann factors by using the energetic and thermodynamic information obtained during the quantum-chemical calculations, in particular before simulating the NMR spectra.

Advantageously, calculations after classifying the graphs, in particular quantum-chemical calculations, can be optimized and only performed for the non-equivalent sets encompassing distinguishable conformer molecular structures, since the physical properties are identical for the molecular structures within one individual equivalent set or can be averaged over all molecular structures within one individual equivalent set, for example in case of NMR spectroscopy, by using permutational mappings. The permutational mappings are preferably the atom mappings assigning the rotamers to the representative molecular structure and to each other and in other words grouping the rotamers into the equivalent sets.

In a preferred embodiment, apparatus comprises a thermodynamic simulation unit for simulating thermodynamic properties from the plurality of equivalent sets.

In a preferred embodiment, the apparatus comprises a chemical reaction simulation unit for simulating chemical reactions from the plurality of equivalent sets.

Favorably, the present invention can be seamlessly integrating into existing computational workflows to enhance the ensemble of relevant molecular conformations. This results in more accurate predictions of for example NMR spectra, thermodynamic properties or chemical reactions, leading to predictions is better alignment with experimental data.

Additionally, when analyzing a sample of unknown composition with several possible molecular components, simulating the spectra of these molecules can provide insights into the identity of the compounds or even the composition of a sample mixture. Likewise, the expected outcome of a chemical synthesis can be confirmed by comparing the experimental NMR spectrum with the simulated spectrum. Similarly, the approach according to the present invention can beneficially be applied in the analysis of complex biological samples, such as for example urine, to determine their composition or to identify potential drugs or metabolites.

In a preferred embodiment, the interface is configured for receiving input data relating to an incomplete set of permutations. In another preferred embodiment, the processing unit is configured for (a) converting the incomplete set of permutations into a plurality of permutation matrices, wherein each permutation matrix preferably represents one individual molecular structure. In particular, the processing unit is configured for (b) generating a new permutation matrix by multiplying one individual permutation matrix of the plurality of permutation matrices with another individual permutation matrix of the plurality of permutation matrices or with itself. Preferably, the processing unit is configured for (c) comparing the new permutation matrix with the plurality of permutation matrices for determining if the new permutation matrix is identical or not identical to any other individual permutation matrix of the plurality of permutation matrices. Beneficially, the processing unit is configured for (d) including the new permutation matrix into the plurality of permutation matrices, if the new permutation matrix is not identical to any other individual permutation matrix of the plurality of permutation matrices. Advantageously, the processing unit is configured for (e) repeating (b) to (d), at least until all possible different multiplications within the plurality of permutation matrices, including the multiplications of individual permutation matrices with themselves, have been performed.

In a preferred embodiment, the method comprising steps of receiving input data relating to an incomplete set of permutations. Preferably, the method comprising steps of (a) converting the incomplete set of permutations into a plurality of permutation matrices, wherein each permutation matrix preferably represents one individual molecular structure. In particular, the method comprising steps of (b) generating a new permutation matrix by multiplying one individual permutation matrix of the plurality of permutation matrices with another individual permutation matrix of the plurality of permutation matrices or with itself. Advantageously, the method comprising steps of (c) comparing the new permutation matrix with the plurality of permutation matrices for determining if the new permutation matrix is identical or not identical to any other individual permutation matrix of the plurality of permutation matrices. Beneficially, the method comprising steps of (d) including the new permutation matrix into the plurality of permutation matrices if the new permutation matrix is not identical to any other individual permutation matrix of the plurality of permutation matrices. Preferably, the method comprising steps of (e) repeating (b) to (d), at least until all possible different multiplications within the plurality of permutation matrices, including the multiplications of individual permutation matrices with themselves, have been performed.

Said embodiments are based on the idea of using the multiplication of permutation matrices to complete the set of rotamers for a given conformer ensemble, in particular for each individual equivalent set. The input data is preferably based on an incomplete set of permutations, which are also referred to as the permutational mappings, possibly originating from - but not restricted to - the molecular structures sorted by their equivalence in the equivalent sets. In particular, the input data is representing the atom permutations between the different rotamers. Advantageously, the current concept is able to determine all valid permutations and thereby complete an incomplete rotamer ensemble. This allows for the usage of improved conformer ensembles and/or rotamer ensembles for example for an improved simulation of NMR spectra, which match more accurate with experimentally measured NMR spectra.

In a preferred embodiment, the processing unit is configured for (e) repeating (b) to (d), until no new permutation matrix is added to the plurality of permutation matrices. In particular, this repeating loop is continued as long as required. Thereby, the probability of incomplete data can be reduced drastically.

In a preferred embodiment, the interface is configured for receiving input data relating to the incomplete set of permutations representing the plurality of equivalent sets of molecular structures. Preferably, the permutational mappings are converted into permutation matrices for example by employing the Python library NumPy^{®} (https://numpy.org/). The resulting permutation matrices are then preferably represented by NumPy^{®} arrays. The multiplication of two individual permutation matrices results in the generation of a new permutation matrix. In particular, if the multiplication is repeatedly applied on an individual permutation matrix, the identical permutation is obtained, in other words, the original molecular structure is obtained. In other words, repeated multiplication of an individual permutation matrix with itself in particular ultimately results in a unit matrix. Favorably, when all possible different multiplications between the permutation matrices of the set of permutations, in particular of the incomplete set of permutations, are performed, the resulting new permutation matrix or the plurality of new permutation matrices arising from a plurality of multiplication operations, can be either already present in the input set of permutation matrices, or if not, the new permutation matrix or the plurality of new permutation matrices are added to the plurality of permutation matrices.

Favorably, the current concept also includes the multiplication of every individual permutation matrix with itself for generating new permutation matrices. If the new permutation matrix is not identical to any other individual permutation matrix of the plurality of permutation matrices, it is not yet part of the plurality of permutation matrices and is therefore included in the plurality of permutation matrices for completing the plurality of permutation matrices and preferably this new permutation matrix is then representing a molecular structure which was not part of the plurality of molecular structures included before. Advantageously, as previously mentioned, since a repeated multiplication of permutation matrices results in the unit matrix at some point, only a finite number of new permutations matrices can be found.

Preferably, after including the new permutation matrix or the plurality of new permutation matrices into the plurality of permutation matrices, the multiplication is repeated with the extended plurality of permutation matrices comprising the new permutation matrix or the plurality of new permutation matrices. In particular, every new permutation matrix is also multiplied with itself. The current concept is beneficially repeated with the extended set until no new permutation matrices are obtained anymore.

In a preferred embodiment, the interface is configured for receiving input data, wherein each individual equivalent set comprises at least one non-trivial atom permutation. Preferably, at least one non-trivial atom permutation per symmetric group is provided in the input data. Advantageously, the complete ensemble of rotamers is obtained in the end.

In a preferred embodiment, the apparatus is further configured for classifying the plurality of molecular structures into the plurality of equivalent sets, which is represented by (and/or corresponds to) the incomplete set of permutations, wherein: the interface is configured for receiving input data relating to the molecular structures to be classified and at least one tolerance parameter for setting a tolerance, the processing unit is configured for
- generating a plurality of graphs from the plurality of molecular structures, wherein preferably for each individual molecular structure of the plurality of molecular structures one graph is generated,
- comparing each individual graph from the plurality of graphs to all other individual graphs,
- determining whether the graphs are equivalent or non-equivalent within the tolerance based on the tolerance parameter, and
- classifying the graphs into the plurality of equivalent sets, which is represented by the incomplete set of permutations, wherein each individual equivalent set of the plurality of equivalent sets is non-equivalent to all other equivalent sets.

Favorably, the current concept allows transferring rotamer atom mappings between different conformers. In particular, if two conformers are in a thermodynamic equilibrium, it is possible for a first conformer to rearrange into a second conformer, then undergo a transition to a different rotamer of the second conformer, and rearrange back to the first conformer, thus obtaining a different rotamer of first conformer. In other words and in particular, a first individual molecular structure from a first equivalent set can rearrange into a second individual molecular structure of a second equivalent set. Thereby, sets of atom mappings of different conformers are combined, in particular by performing all matrix multiplications of the plurality of permutation matrices. Thus, the completeness of rotamer ensembles, especially of energetically higher-lying conformers, can be beneficially obtained, which are otherwise sampled less well than the lower-lying conformers in molecular dynamics or metadynamics calculations.

Preferably, the steps of the method or the methods are carried out consequently in the order mentioned in the claims or according to the description, if not discrepant to the claims. The methods disclosed herein are preferably computer implemented and can particularly be implemented in the form of the scientific software running on a personal computer.

These and other aspects of the invention will be apparent from and elucidated with reference to the embodiment(s) described hereinafter. In the following drawings
- Figure 1: shows a schematic illustration of an apparatus according to the present invention;
- Figure 2: shows a more detailed schematic illustration of an apparatus according to the present invention;
- Figure 3: shows a schematic illustration of an apparatus for completing a set of permutations;
- Figure 4: shows a more detailed schematic illustration of an apparatus for completing a set of permutations; and
- Figure 5: shows a detailed schematic illustration of an apparatus according to the present invention, which is also capable of completing a set of permutations for simulating NMR spectra.

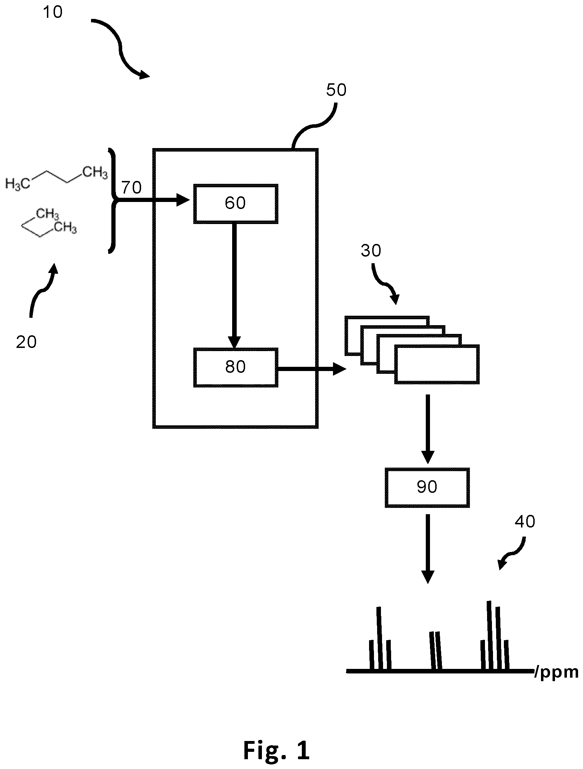

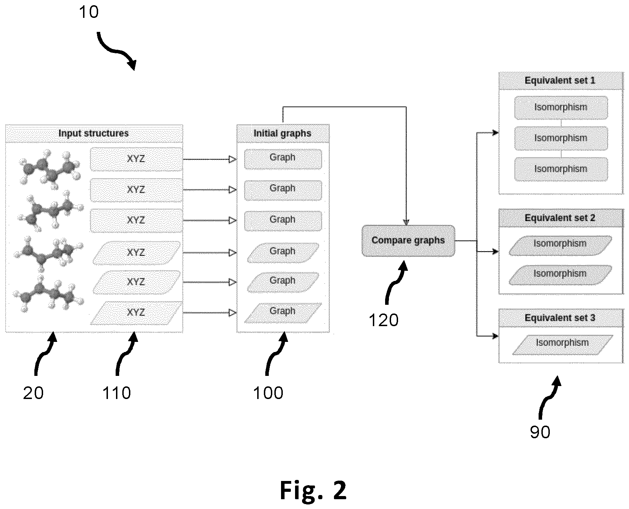

Fig. 1 shows a schematic illustration of an apparatus 10 according to the present invention. The apparatus 10 is suitable for classifying a plurality of molecular structures 20 into a plurality of equivalent sets 30. This can be particularly useful for simulating NMR spectra 40. The apparatus 10 can be implemented in the form of a computer or a software running on a computer. In the illustrated example and in all following examples, the apparatus 10 is implemented in the form of a computer, comprising and running a scientific simulation software 50, which comprises an interface 60 for receiving input data 70 relating to the molecular structures 20 to be classified. The interface 60 is further configured for receiving at least one tolerance parameter for setting a tolerance. The processing unit 80 is configured for generating a plurality of graphs 100 (see Fig. 2) from the plurality of molecular structures 20. For each individual molecular structure of the plurality of molecular structures 20 one graph is generated. The processing unit 80 is also configured for comparing 120 (see Fig. 2) each individual graph from the plurality of graphs 100 to all other individual graphs. In addition, the processing unit 80 is configured for determining whether the graphs are equivalent or non-equivalent within the tolerance based on the tolerance parameter and for classifying the graphs 100 into the plurality of equivalent sets 30, wherein each individual equivalent set of the plurality of equivalent sets 30 is non-equivalent to all other equivalent sets 30.

Fig. 2 shows a more detailed schematic illustration of an apparatus 10 according to the present invention. The input molecular structures 20 comprise structural parameters 110, which are provided in a XYZ format. The input molecular structures 20 can be generated by a conformer search software e.g. by RDKit^{™} or by CREST^{™}, or by performing a molecular dynamics simulation. Preferably, the XYZ format includes the atom composition and the molecular structure parameters 110 as coordinates, which are then converted into the molecular graphs 100. Subsequently, a first individual graph from the plurality of graphs 100 is taken as a reference and compared 120 to all other graphs of the plurality of graphs 100. If all structural parameters such as for example the bond lengths, the bond angles, and the dihedral angles are within the tolerance as indicated by the tolerance parameter, the structures are determined as equivalent, in particular irrespective of the atom ordering in the XYZ input. If the structures are equivalent, all possible isomorphisms, such as for instance permutations needed to map one structure to the other, are returned classified together in an equivalent set 30, in particular with the respective index of the molecular structures. This procedure is preferably repeated with the remaining graphs from the plurality of graphs 100. This loop can continue as long as required. The equivalent molecular structures are beneficially classified again into the respective equivalent sets 30 together with their isomorphisms, and the remaining molecular structures are compared again, until all graphs 100 and thus all molecular structures are completely classified and sorted into the equivalent sets 30. Favorably, one individual graph, representing one molecular structure is chosen as a representative molecular structure from each equivalent set. In particular, the molecular structures that were identified as equivalent are collected, preferably with their reference and their isomorphisms or rotamers. As a result, the non-equivalent conformer molecular structures are advantageously grouped together with the permutational mappings for their rotamers. The output valuably provides a plurality of equivalent sets 30, wherein the equivalent sets 30 represent non-equivalent and thus distinguishable molecular structures. One distinct equivalent set beneficially comprises one or a plurality of molecular structures, with each of those having a list of isomorphisms representing rotamers. Preferably, the current invention thus gives access to the number of equivalent structures per conformer and the atom mapping of one rotamer to another. If provided in the input data, energetic information of the different molecular structures is preserved. The output is for example compatible with the data validation Python package Pydantic^{®} (https://docs.pydantic.dev) and other standard formats. The output may be validated by the Python package Pydantic^{®}.

The input molecular structures (20) may be received in various formats. The most common situation would be a representation in "XYZ" format, comprising the chemical element symbol and the atomic coordinates (x, y and z) of each atom. It may then be required that in the step of generating the plurality of graphs, i.e. the generation of the graph representation, this input is converted into a molecular geometry representation (internal coordinates) with bond lengths, angles and dihedrals. In an exemplary implementation, this may, e.g. be achieved via the following steps:
(a) First, bonds between atoms are identified. For this purpose, the interatomic distances of each pair of atoms may be compared with a threshold. This threshold is preferably determined with the help of the covalent radii of the respective atoms, more preferably as 1.2 times the sum of the two respective covalent radii. The atoms are assumed to share a bond if their distance is below the threshold. The bonding topologies may also be determined in other ways.
(b) Unless already done in the previous step, distances are calculated for all bonded atom pairs.
(c) Angles are calculated for all atom triples A-B-C if atoms A-B and atoms B-C share bonds.
(d) Dihedrals are calculated for all atom quadruples A-B-C-D if atom pairs A-B, B-C and C-D share bonds.

It is to be understood that if the input molecular structures (20) are already made available in a format that, in contrast to the XYZ format, contains information on the bonding topology or that contains bond lengths, angles or dihedrals, then the respective steps above become irrelevant.

In the step of generating the plurality of graphs a graph representing the molecular structure may particularly be stored as an undirected graph (e.g. by using the NetworkX library).
(a) For each atom, a node is added to the graph object with the chemical element of the respective atom stored as node information.
(b) For each bond, an edge is added to the graph between the nodes representing the two atoms. The interatomic distance is added to the edge information.

Angles and dihedrals are quantities that involve three or four atoms. Ideally, a graph library would natively support storing and processing of information concerning three of four nodes at once. Since NetworkX only supports edges as information concerning pairs of nodes, but no "edges" between three or four nodes, workarounds may be needed in an implementation. These workarounds may particularly include the following steps:
(a) For each angle, an edge is added between the terminal atoms: for a triple of atoms A-B-C with an angle, an edge is added between atoms A and C, regardless of whether they share a bond. An edge can represent information on at most one bond length and any number of angles or dihedrals. The value of the angle A-B-C is added as information to the edge A-C together with the triple of the atom indices: In a preferred implementation the information can be stored in a dictionary with the atom triples as keys and the angles as values.
(b) For each dihedral, an edge is added between the terminal atoms. For a quadruple of atoms A-B-C-D, an edge is added between atoms A and D, regardless of whether they share a bond. The value of the dihedral A-B-C-D is added as information to the edge A-D together with the quadruple of atom indices: In a preferred implementation, the information can be stored in a dictionary with the atom quadruples as keys and the dihedrals as values.

Thus, information for each edge between nodes representing atoms A and B can be structured as follows:

```
 {
   "distance": value or None
   "angle": {
     (index A, index X, index B): value,
   }
   "dihedral": {
     (index A, index X, index Y, index B): value,
   }
   }
```

In the step of comparing each individual graph to the other graphs, it may particularly be determined whether or not the graphs are isomorphic. The NetworkX library, e.g., implements a functionality to determine if graphs are isomorphic: This is the case if the graphs can be mapped onto one another such that their node and edge topologies match, and such that the respective node and edge information satisfies arbitrary criteria defined by the programmer making use of the NetworkX library. The programmer is required to implement functions that accept either a pair of nodes from both graphs or a pair of edges from both graphs as input arguments, and return a truth value indicating if the information matches.

Functions performing edge comparisons can particularly be implemented as follows:
(a) Edges match only if the distances of the edge of the first graph and the edge of the second graph agree to within a tolerance, or if distances are absent in both edges.
(b) Edges match only if they have the same number of angles, and if a one-to-one relationship can be established of angle values from the one edge with angle values from the other edge to within a numerical tolerance criterion. In a preferred implementation, the tolerance criterion may be that |*e*^{*iϕ*₁} - *e*^{*iϕ*₂}| needs to be below a tolerance, with *ϕ*₁ and *ϕ*₂ representing the respective two angles in radians.
(c) Edges match only if they have the same number of dihedrals, and if a one-to-one relationship can be established of dihedral values from the one edge with dihedral values from the other edge to within a numerical tolerance criterion. In a preferred implementation, the tolerance criterion may be that |*e*^{*iθ*₁} - *e*^{*iθ*₂}| needs to be below a tolerance, with *θ*₁ and *θ*₂ representing the respective two dihedrals in radians.

The procedure outlined above can falsely identify isomorphisms that are no isomorphisms upon more rigorous checking. Therefore, a preferred implementation may include an additional step to remove false isomorphisms from the solution. This screening step may thereby map all atom index triples representing angles between the two graphs, verify if each thus uniquely identified pair of angle values satisfies the numerical tolerance criterion, map all atom index quadruples representing dihedrals between the two graphs, and verify if each thus uniquely identified pair of dihedral values satisfies the numerical tolerance criterion.

The step of determining whether the graphs are equivalent may particularly correspond to a selection of isomorphisms. In an implementation based on the NetworkX library multiple isomorphisms between molecules structure graphs may be identified (if they exist). For applications such as averaging NMR parameters over the rotamers associated with a conformer, a single isomorphism per rotamer may need to be chosen. Preferably, an isomorphism *m*(*i*) is chosen that maps atom indices *i* of one structure onto atom indices of the other structure such that Σᵢ|*m*(*i*) - *i*| is minimized. Alternatively, one could choose a root-mean-square-deviation or another criterion.

Since nuclear magnetic resonance spectroscopy is insensitive to the overall chirality, efficiency gains are made by reusing computed information for mirror images of molecular structures where they are represented in the structural ensemble. To include mirror images in the structure identification, a preferred implementation may include an option that permits comparing the three-dimensional structure of the one molecule with the inverted structure of the other molecule, in addition to comparing the two original structures.

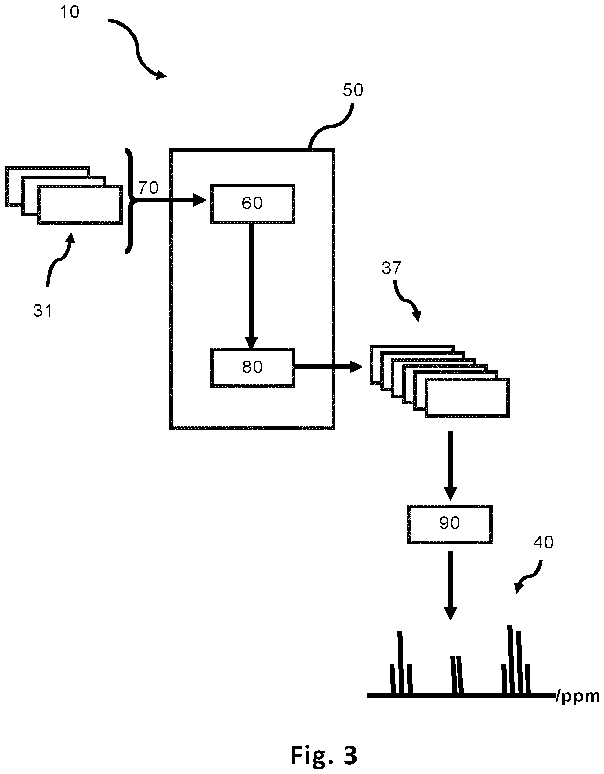

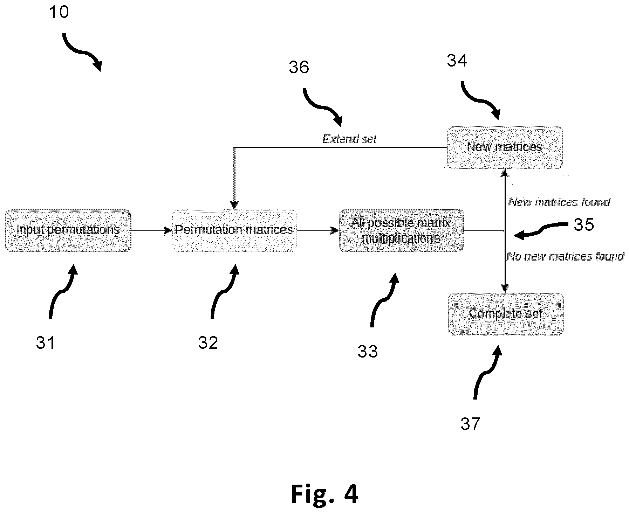

Fig. 3 shows a schematic illustration of an apparatus 10 for completing a set of permutations 31. The input set of permutations 31 is representing an incomplete plurality of equivalent sets 30 of molecular structures. The apparatus 10 is capable of completing a set of permutations 31. The apparatus 10 is for example employed for simulating NMR spectra 40, with a scientific simulation software 50. After completing, a complete set of permutations 37 is yielded. The completed set of permutations 37 is thus representing a completed plurality of equivalent sets of molecular structures. The scientific simulation software 50 comprises an interface 60 for receiving input data 70 relating to an incomplete set of permutations 31. The scientific simulation software 50 further comprises a processing unit 80 for converting the incomplete set of permutations 31 into a plurality of permutation matrices 32 (see Fig. 4), wherein each permutation matrix preferably represents one individual molecular structure. The processing unit 80 is further configured for multiplying 33 (see Fig. 4) two individual permutation matrices of the plurality of permutation matrices 32 which each other to generate a new permutation matrix 34 (see Fig. 4). Moreover, the processing unit 80 is configured for multiplying 33 (see Fig. 4) one individual permutation matrix with itself for generating a new permutation matrix 34 (see Fig. 4). Also, the processing unit 80 is configured for comparing 35 (see Fig. 4) the new permutation matrix 34 with the plurality of permutation matrices 32, for determining if the new permutation matrix 34 is identical or not identical to any other individual permutation matrix of the plurality of permutation matrices 32.

Fig. 4 shows a more detailed schematic illustration of an apparatus 10 for completing a set of permutations 31. In the present example a molecule with a single methyl group and thus three equivalent hydrogen atoms is illustrated, namely acetaldehyde (H₃CCHO). The hydrogen atoms of the methyl group are labeled 0, 1, and 2. In particular, there are three different possible rotations or permutations. In the present example CREST^{™} generated only two different rotamers, with a first rotamer one corresponding to a trivial permutational mapping a = {0: 0, 1: 1, 2: 2}, which is also referred to as the identity, and a second rotamer corresponding to a non-trivial permutational mapping: b = {0: 1, 1: 2, 2: 0}. The permutational mappings can be converted to permutation matrices. In the present discussion the function of the software 50 is explained with the permutational mappings, which is identical to the function with permutation matrices. Multiplying 33 the identity a = {0: 0, 1: 1, 2: 2} of the first rotamer with itself, gives the same trivial permutational mapping again (a∘a = a). Also, multiplying 33 the identity representing the first rotamer with the non-trivial permutational mapping representing the second rotamer gives the same permutational mapping of the second rotamer again (a∘b = b∘a = b). However, multiplying 33 the non-trivial permutational mapping of the second rotamer with itself (b∘b = c) gives a new non-trivial permutational mapping: c = {0: 2, 1: 0, 2: 1}. This new non-trivial permutational mapping represents a third rotamer and is thus included 36 in the plurality of permutational mappings. Multiplying the new non-trivial permutational mapping of the third rotamer c = {0: 2, 1: 0, 2: 1} with itself results in the non-trivial permutational mapping of the second rotamer (c∘c = b). Multiplying 33 the new non-trivial permutational mapping of the third rotamer c = {0: 2, 1: 0, 2: 1} with the identity results in the same non-trivial permutational mapping c of the third rotamer (a∘c = c∘a = c). Multiplication 33 of the non-trivial permutational mapping of the second rotamer b with the non-trivial permutational mapping of the third rotamer c results in the identity permutational mapping of the first rotamer (b∘c = c∘b = a), thereby demonstrating that the set of permutational mappings is complete. The same steps can be performed with the plurality of permutation matrices accordingly.

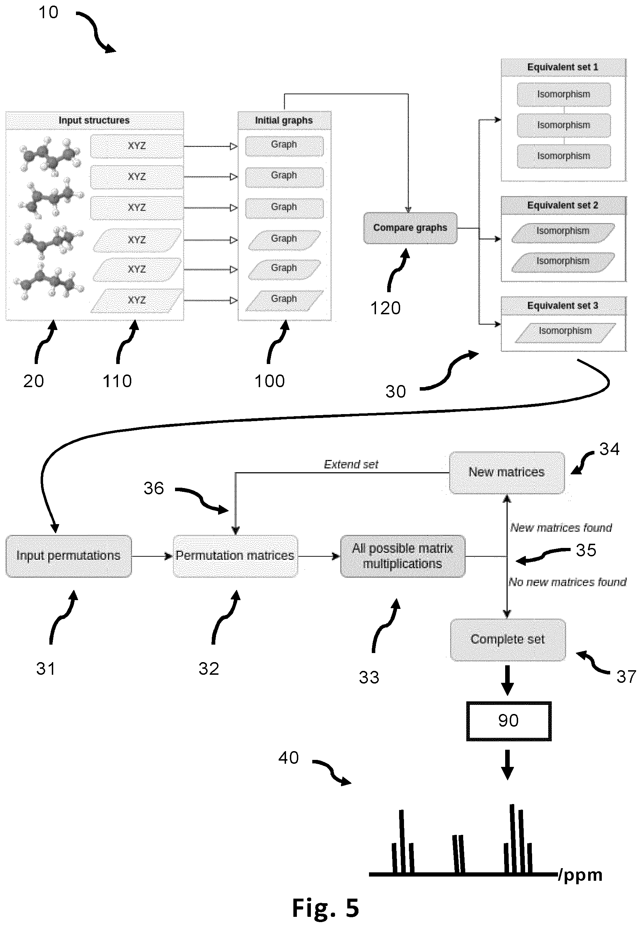

Fig. 5 shows a detailed schematic illustration of an apparatus 10 according to the present invention capable of completing a set of permutations for simulating NMR spectra 40. The apparatus 10 is firstly employed for classifying a plurality of molecular structures 20 into a plurality of equivalent sets 30 and subsequently for completing a set of permutations 31, which corresponds to the plurality of equivalent sets 30. The apparatus 10 comprises a scientific simulation software 50 with an interface 60 (see Fig. 1 and 2) for receiving input data 70 (see Fig. 1 and 2) relating to the molecular structures 20 to be classified and at least one tolerance parameter for setting a tolerance. The processing unit 80 is configured for generating a plurality of graphs 100 from the plurality of molecular structures 20, wherein preferably for each individual molecular structure of the plurality of molecular structures 20 one graph is generated. The processing unit 80 is configured for comparing 120 each individual graph from the plurality of graphs 100 to all other individual graphs, and determining whether the graphs are equivalent or non-equivalent within the tolerance as indicated by the tolerance parameter, and classifying the graphs 100 into the plurality of equivalent sets 30, wherein each individual equivalent set of the plurality of equivalent sets 30 is non-equivalent to all other equivalent sets 30. The equivalent sets 30 are equal to the incomplete set of permutations 31. The interface 60 (see Fig. 1 and 2) is further configured for receiving input data 70 relating to the incomplete set of permutations 31. In other words, the interface 60 is receiving the equivalent sets 30. The processing unit 80 is further configured for (a) converting the incomplete set of permutations 31 into a plurality of permutation matrices 32, wherein each permutation matrix preferably represents one individual molecular structure. The processing unit 80 is also configured for (b) generating a new permutation matrix 34 by multiplying 33 one individual permutation matrix of the plurality of permutation matrices 32 with another individual permutation matrix of the plurality of permutation matrices 32 or with itself. In addition, the processing unit 80 is configured for (c) comparing 35 the new permutation matrix 34 with the plurality of permutation matrices 32 for determining if the new permutation matrix 34 is identical or not identical to any other individual permutation matrix of the plurality of permutation matrices 32. The processing unit 80 is configured for (d) including 36 the new permutation matrix 34 into the plurality of permutation matrices 32, if the new permutation matrix 34 is not identical to any other individual permutation matrix of the plurality of permutation matrices 32. The processing unit 80 is also configured for (e) repeating (b) to (d), at least until all possible different multiplications 33 within the plurality of permutation matrices 32, including the multiplications of individual permutation matrices with themselves, have been performed.

The disclosed invention is advantageously applied for the simulation of NMR spectra 40, and can be seamlessly integrating into existing computational workflows to complete a conformer and rotamer ensemble as explained previously. In the following, an example of how the apparatus 10 and method, of the present invention can be put to use are described. The example relates to simulations of NMR spectra 40 with an NMR spectrum simulation unit 90. Using a complete conformer ensemble as equivalent set 37 leads to an improved prediction of NMR spectra 40 as the results are in better alignment with the experimentally measured NMR spectra. In the following, an exemplary workflow developed will be described:
Starting from a single input initial molecular structure, which may be provided as a 2D structure in a so-called SMILES string, or simply as a name associated with the molecular structure. In case the molecular structures name is provided, the SMILES string is obtained e.g. via a database such as the PubChem^{®} database. The input 2D molecular structure is then converted into an initial 3D structure using the RDKit^{™} or Open Babel^{™} software. With this 3D structure as a starting point, an initial geometry optimization may be performed. Next, CREST^{™} is used to generate a plurality of molecular structures 20. From this output, only the data relating to the molecular structures 20 is used, while the assignment of conformers and rotamers provided by CREST^{™} is ignored. The entire structural ensemble is grouped into conformers and rotamers using the apparatus 10 and/or method for classifying a plurality of molecular structures. Afterwards, the obtained classified equivalent set 20, in other words the classified conformer and rotamer ensemble, which is likely to be incomplete, is completed by using the apparatus 10 and/or method for completing a set of permutations 31. After completing the ensemble of conformer and rotamer molecular structures, quantum-chemical calculations are performed on the representative conformer structures to obtain energies and NMR parameters, for example by the NMR spectrum simulation unit 90. For each equivalent set of conformers, in other words for each equivalent set 30, the NMR parameters are averaged over the rotamers comprised in an individual equivalent set by using the atom mappings obtained during the procedure, and the different conformers are weighted according to their Boltzmann factors by using the energetic and thermodynamic information obtained during the quantum-chemical calculations. This data is then utilized by the NMR spectrum simulation unit 90 for simulating the NMR spectrum 40.

In summary, the methods and apparatus of the present invention described herein offer a novel approach to improving the efficiency of simulation software, in particular in the fields of NMR simulations.

The foregoing discussion discloses and describes merely exemplary embodiments of the present disclosure. As will be understood by those skilled in the art, the present disclosure may be embodied in other specific forms without departing from the spirit or essential characteristics thereof. Accordingly, the description is intended to be illustrative, but not limiting the scope of the disclosure, as well as other claims. The disclosure, including any readily discernible variants of the teachings herein, defines, in part, the scope of the foregoing claim terminology such that no inventive subject matter is dedicated to the public.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

In so far as embodiments of the disclosure have been described as being implemented, at least in part, by an apparatus, which is in particular a software-controlled data processing apparatus, it will be appreciated that a non-transitory machine-readable medium carrying such software, such as an optical disk, a magnetic disk, semiconductor memory or the like, is also considered to represent an embodiment of the present disclosure. Further, such software may also be distributed in other forms, such as via the Internet or other wired or wireless telecommunication systems. A method according to the present invention may particularly be carried out to control the operation of a software defined radio.

The elements and features of the disclosed units, apparatus, and devices may be implemented by corresponding hardware and/or software elements, for instance appropriated circuits. A circuit is a structural assemblage of electronic components including conventional circuit elements, integrated circuits including application specific integrated circuits, standard integrated circuits, application specific standard products, and field programmable gate arrays. Further a circuit includes central processing units, graphics processing units, and microprocessors which are programmed or configured according to software code. A circuit does not include pure software, although a circuit includes the above-described hardware executing software.

**List of reference signs**

| | |
|---|---|
| Apparatus | 10 |
| Molecular structures | 20 |
| Equivalent sets | 30 |
| Incomplete set of permutations | 31 |
| Permutation matrices | 32 |
| Multiplying | 33 |
| New permutation matrix | 34 |
| Comparing permutation matrix | 35 |
| Including | 36 |
| Completed set | 37 |
| NMR spectrum | 40 |
| Scientific simulation software | 50 |
| Interface | 60 |
| Input data | 70 |
| Processing unit | 80 |
| NMR spectrum simulation unit | 90 |
| Plurality of graphs | 100 |
| Structural parameters | 110 |
| Comparing each individual graph | 120 |

## Claims

1. Apparatus (10) for classifying a plurality of molecular structures (20) into a plurality of equivalent sets (30), for example for simulating NMR spectra (40) and/or thermodynamic properties and/or chemical reactions, comprising:
an interface (60) for receiving input data (70) relating to the molecular structures (20) to be classified and at least one tolerance parameter for setting a tolerance,
a processing unit (80) for
- generating a plurality of graphs (100) from the plurality of molecular structures (20), wherein preferably for each individual molecular structure of the plurality of molecular structures (20) one graph is generated,
- comparing (120) each individual graph from the plurality of graphs (100) to all other individual graphs,
- determining whether the graphs are equivalent or non-equivalent within the tolerance based on the tolerance parameter, and
- classifying the graphs (100) into the plurality of equivalent sets (30), wherein each individual equivalent set of the plurality of equivalent sets (30) is non-equivalent to all other equivalent sets (30).

2. Apparatus (10) according to the preceding claim, wherein the processing unit (80) is configured for classifying the graphs (100) into the plurality of equivalent sets (30), wherein each individual equivalent set of the plurality of equivalent sets (30)
- comprises either one single graph or a plurality of graphs representing rotamer molecular structures,
- comprises either one single graph or a plurality of graphs representing molecular structures with equivalent bond topologies, and/or
- is representing a non-equivalent conformer molecular structure in comparison to the molecular structures of all other equivalent sets.

3. Apparatus (10) according to any preceding claim, wherein the interface (60) is configured for receiving input data (70) relating to the molecular structures (20), wherein the input data (70) comprises an atom composition and structural parameters (110) such as coordinates of atoms and/or bond lengths and/or bond angles and/or dihedral angles.

4. Apparatus (10) according to any preceding claim, wherein
the interface is configured for receiving input data (70) including atomic coordinates of each atom of the molecular structures (20), and
the processing unit (80) is configured for generating the plurality of graphs (100) from the plurality of molecular structures (20) by determining a molecular geometry representation with bond lengths, angles and dihedrals based on the atomic coordinates.

5. Apparatus (10) according to any preceding claim, wherein
the processing unit (80) is configured for comparing (120) each individual graph from the plurality of graphs (100) to all other individual graphs based on a determination whether node and edge topologies match and the graphs are isomorphic, and
the processing unit (80) is preferably configured for removing falsely identified isomorphisms.

6. Apparatus (10) according to any preceding claim, wherein the processing unit (80) is configured for determining whether the graphs are equivalent or non-equivalent based on a selection of isomorphisms.

7. Apparatus (10) according to any preceding claim, comprising:
a conformation generating unit for generating the input data (70) relating to the molecular structures (20), wherein the molecular structures (20) comprise a plurality of non-equivalent conformer molecular structures and rotamer molecular structures,
wherein the interface (60) preferably comprises a computer interface for receiving the input data (70) relating to the plurality of molecular structures (20) and a user interface for receiving an initial molecular structure, and wherein the conformation generating unit is preferably configured for generating the input data (70) from the initial molecular structure.

8. Apparatus (10) according to the preceding claim, wherein the user interface is configured for receiving the initial molecular structure as a structural formula, in particular a 2D structural formula, or as 3D data, in particular X-ray diffraction data.

9. Apparatus (10) according to any preceding claim, wherein the interface (60) is configured for receiving the tolerance parameter relating to a bond length tolerance and/or a bond angle tolerance and/or a dihedral angle tolerance, wherein
the bond length tolerance and/or the bond angle tolerance and/or the dihedral angle tolerance are each from 0.1% to 10%, preferably from 0.1% to 5%, more preferably from 1% to 3%,
the bond length tolerance is from 1 pm to 10 pm, preferably from 1 pm to 5 pm, more preferably from 2 pm to 3 pm,
the bond angle tolerance is from 0.1 ° to 10.0°, preferably from 0.2° to 5.0°, more preferably 0.5°, and/or
the dihedral angle tolerance is from 0.1° to 10.0°, preferably from 0.2° to 5.0°, more preferably 0.5°.

10. Apparatus (10) according to any preceding claim, comprising:
an NMR spectrum simulation unit (90) for simulating an NMR spectrum (40) from the plurality of equivalent sets (30), and/or
a thermodynamic simulation unit for simulating thermodynamic properties from the plurality of equivalent sets (30).

11. Apparatus (10) according to any preceding claim, further configured for completing an incomplete set of permutations (31) corresponding to the plurality of equivalent sets (30) of molecular structures, wherein:
the interface (60) is configured for receiving input data (70) relating to the incomplete set of permutations (31),
the processing unit (80) is configured for
(a) converting the incomplete set of permutations (31) into a plurality of permutation matrices (32), wherein each permutation matrix preferably represents one individual molecular structure,
(b) generating a new permutation matrix (34) by multiplying (33) one individual permutation matrix of the plurality of permutation matrices (32) with another individual permutation matrix of the plurality of permutation matrices (32) or with itself,
(c) comparing (35) the new permutation matrix (34) with the plurality of permutation matrices (32) for determining if the new permutation matrix (34) is identical or not identical to any other individual permutation matrix of the plurality of permutation matrices (32),
(d) including (36) the new permutation matrix (34) into the plurality of permutation matrices (32), if the new permutation matrix (34) is not identical to any other individual permutation matrix of the plurality of permutation matrices (32),
(e) repeating (b) to (d), at least until all possible different multiplications (33) within the plurality of permutation matrices (32), including the multiplications of individual permutation matrices with themselves, have been performed.

12. Method for classifying a plurality of molecular structures (20) into a plurality of equivalent sets (30), for example for simulating NMR spectra (40) and/or thermodynamic properties and/or chemical reactions, comprising steps of:
- receiving input data (70) relating to the molecular structures (20) to be classified and at least one tolerance parameter for setting a tolerance,
- generating a plurality of graphs (100) from the plurality of molecular structures (20), wherein preferably for each individual molecular structure of the plurality of molecular structures one graph is generated,
- comparing (120) each individual graph from the plurality of graphs (100) to all other individual graphs,
- determining whether the graphs are equivalent or non-equivalent within the tolerance based on the tolerance parameter, and
- classifying the graphs (100) into the plurality of equivalent sets (30), wherein each individual equivalent set of the plurality of equivalent sets (30) is non-equivalent to all other equivalent sets (30).

13. A computer program comprising instructions which, when the program is executed by a computer, cause the computer to carry out the method of the preceding claim.

14. A data carrier signal carrying the computer program of the preceding claim.

15. A computer-readable medium comprising instructions which, when executed by a computer, cause the computer to carry out the method of claim 12.
